# EUROPÄISCHE PATENTANMELDUNG

(11) **EP 2 336 783 A1**
(43) Veröffentlichungstag der Anmeldung: **22.06.2011**
(21) Anmeldenummer: 09179898.3
(22) Anmeldetag: 18.12.2009
(51) Int. Cl.: G01N 33/68

(54) **C-terminale Sequenzierung von Aminosäuren**

(71) Anmelder: BASF SE, 67056 Ludwigshafen (DE)
(72) Erfinder: Brugger, Thomas, 67127 Rödersheim-Gronau (DE); Friedrich, Thomas, 64283 Darmstadt (DE)

(57) **Zusammenfassung**

Die vorliegende Erfindung leistet einen wertvollen Beitrag zur Sequenzbestimmung des C-Terminus einer Aminosäuresequenz, wofür es bislang keine zuverlässig einsetzbaren Methoden gibt. Das erfindungsgemäße Verfahren ist dadurch gekennzeichnet ist, dass man die freien COOH-Gruppen im Protein mit Marker A kennzeichnet, das markierte Protein in Peptide spaltet, die neu freigewordenen COOH-Gruppen direkt oder indirekt dazu nutzt, das C-terminale Peptidfragment von den anderen Peptidfragmenten zu trennen und zu identifizieren, um dieses im Anschluss in üblicher Weise zu sequenzieren.

## Beschreibung

Die vorliegende Erfindung beinhaltet eine Methode zur Aufklärung der C-terminalen Aminosäuresequenz eines Proteins.

Die endständigen funktionellen Gruppen einer Aminosäuresequenz sind einerseits eine alpha-Amino- und andererseits eine alpha-Carboxygruppe. Die sogenannten Termini der Sequenz werden im Speziellen als N- bzw. C-Terminus bezeichnet. Beide Termini erlauben unterschiedliche chemische Methoden zur Analyse der jeweiligen Aminosäuresequenz. Die chemische (nukleophile) Reaktivität der alpha-Aminogruppe des N-terminalen Aminosäurerestes erlaubt einen einfachen Sequenzierungsprozess.
Die N-terminale Sequenzierung basiert auf dem von Edman etablierten Prinzip, dass mit Phenylisothiocyanat (PITC) in basischem Milieu die N-terminale Aminosäure (AS) des Peptids markiert wird. Unter sauren Reaktionsbedingungen wird die Peptidbindung hinter dieser PTCmarkierten N-terminalen Aminosäure gespalten. Das Produkt ist eine Phenylthiohydantoin-Aminosäure (PTH-AS) und das Restpeptid. Dieser Prozess kann mehrfach wiederholt werden. Die PTH-AS und somit die AS-Sequenz lässt sich mittels Chromatographie identifizieren. Nach 20-40 Zyklen sind weitere Wiederholungen schwierig, da z.B. Reste von PTH-AS - die Probelösung so verunreinigen, dass nicht mehr eindeutig ist, welche PTH-AS wann abgespalten wurde.
Der C-Terminus von Aminosäuresequenzen hat sich als für den Sequenzierungsprozess schwer zugänglich erwiesen. Dies liegt an der inhärenten Schwierigkeit, dass die COOH-Gruppe im Gegensatz zur NH₂-Gruppe relativ unreaktiv ist, d.h. weniger nukleophil ist. Es gab bislang verschiedene Herangehensweisen für diese Aufgabenstellung: chemisch (1, siehe dazu auch Bailey 1995, Journal of Chromatography A Vol 705, 9 47-65), physikalisch (2), und enzymatisch (3).
(1a) Die am besten untersuchte Strategie ist die chemische Thiohydantoin (TH) Reaktion, die sich an den Edman-Abbau anlehnt. Probleme bei dieser Methodik: Hohe Temperaturen im Reaktionsablauf können zum Abbau von Aminosäureseitenketten führen, die zur Aktivierung benötigten Reagenzien sind instabil, und im Zuge der TH-Ausbildung kann es zu Racemisierungen kommen.
(1 b) Die Herstellung eines Carboxyamidoderivates am C-terminalen Ende der Aminosäuresequenz, gefolgt von einer Reaktion mit Bis(1,1-Trifluoracetoxy)-iodobenzen , welches wiederum hydrolytisch abgespalten werden kann, ist insofern limitiert, als dass nur 3-6 Zyklen möglich sind, ehe die Reaktion zum Stillstand kommt.
(1 c) Der C-Terminus wird mit Pivoaloylhydroxamid zur Reaktion gebracht: Infolge werden gemäß Lossen-Abbau Hydroxamsäure-Derivate (in der Regel O-acylierte) am Stickstoff deprotoniert und lagern unter Abspaltung des Acyl-Restes in ein Isocyanat um. Die Einschränkung dieser Methode liegt in der Unzugänglichkeit von Asparagin- und Glutaminsäureresten für diese Methode.
(2) Physikalisch werden vor allem FAB/MS (fast atom bombardment mass spectrometry) und NMR (nuclear magnetic resonance) zur C-terminalen Sequenzierung eingesetzt. Nachteilig ist, dass nur 1-10 nanomol Peptid für FAB/MS eingesetzt werden können und die NMR Analyse große Mengen an Peptid benötigt (mikromolar-Bereich).
(3) Die enzymatische Strategie ist dadurch limitiert, dass es schwierig ist, das Ausmaß der Spaltung durch Carboxypeptidasen zu kontrollieren.

Im Folgenden ist der zur Verfügung stehende Stand der Technik zusammengefasst.
US 5,041,388 offenbart eine Methode, bei der das Peptid mit einem Gemisch aus Anhydrid, Isothiocyansäure, und Carbon- oder Kohlensäure unter basischen Bedingungen auf einer aktivierten, festen Oberfläche derivatisiert wird. Das entstehende Peptidyl-Thiohydantoin wird von der festen Phase abgetrennt und auf einer zweiten festen Phase, die das Spaltreagenz trägt weiterreagiert. Das freigesetzte C-terminale Aminoacyl-Thiohydantoin wird abgetrennt und analysiert.
US 5,049,507 lehrt, dass ein Peptid mit einer Mischung aus Anhydrid, Isothiocyansäure, und Carbon-, Kohlen-, oder Sulfonsäure unter basischen Reaktionsbedingungen reagiert, und ein C-terminales Peptidyl-Thiohydantoin entsteht. Die Hydrolyse der C-terminalen Peptidyl-Thiohydantoin-Bindung setzt ein Aminosäure-Thiohydantoin frei, welches über HPLC identifiziert werden kann. Vorzugsweise wird das Peptid über eine interne N-terminale Aminogruppe auf einer soliden Phase immobilisiert und die Reaktion wird über mehrere Runden von C-terminaler Peptidyl-Thiohydantoin-Bildung und Freisetzung von Aminosäure-Thiohydantoin geführt.
Gemäß US 5,059,540 wird ein vorzugsweise auf einer Polyvinyldifluorid-Membran gebundenes Protein mit z. B. Silylisothiocyanat gekoppelt und mit (i) Alkalisalzen niedriger Trialkylsilanolen (z.B. Natrium-Trimethylsilanolat) oder (ii) Trialkylamin-N-oxiden (z.B. Trimethylamin-N-oxid) gespalten. Es werden Spaltreagenzien benötigt, die:
a) Peptidyl-Hydanthoine in volatilen, mit Wasser mischbaren organischen Lösemitteln spalten,
b) eine schnelle und spezifische Reaktion und
c) einen kontinuierlichen Abbau des Peptids vom C-Terminus ausgehend erlauben und
d) das Aminosäure-Hydantoin nicht zerstören. Außerdem
e) soll das Reagenz kein Licht in dem Wellenlängenbereich absorbieren, der für die Detektion des freigesetzten Aminosäure-Thiohydantoin-Derivates verwendet wird.

US 5,066,785 offenbart Kopplungsreagenzien für die C-terminale Aminosäure eines Proteins oder Peptids, nämlich Phosphorylamide oder bevorzugt Phosphorylthioamide. Als Spaltprodukte entstehen dabei Arylhydantoin bzw. Arylthiohydantoin. Es wird auch beschreiben, dass das Peptid vor der Kopplungsreaktion mit Essigsäureanhydrid und Essigsäure aktiviert werden kann.
Die in US 5,304,497 vorgestellte Methode beinhaltet die Immobilisierung eines Peptids auf einer festen Phase über die Aminogruppen, die anschließende Reaktion der N-geschützten Aminosäure mit Halouronium-, Pseudohalouronium-, Tosylphosphonium-, Triflatphosphonium-, oder Sulfonylphosphoniumverbindungen (im nicht-wässrigen Medium), und einem Thiocyanat Reagenz (z.B. Silylisothiocyanat oder ein Kronenetheraddukt von Metallothicyanaten) unter Reaktionsbedingungen, die die Thiohydantoin-Bildung begünstigen. In Folge wird das Aminosäure-Thiohydantoin mit einem Spaltagens hydrolytisch abgetrennt und analysiert.
Die in US 5,468,843 beschriebene Technik umfasst (1) die Behandlung der C-terminalen Aminosäure des Polypeptids mit Essigsäureanhydrid unter Bildung eines Oxozolons, das (2) mit einem Thiocyanatanion unter sauren Bedingungen unter Ausbildung eines Aminosäure-Thiohydantoins reagiert. Es folgt (3) eine Spaltung des Aminosäure-Thiohydantoins vom Polypeptid, und (4) die die Identifizierung desselben. Die Methode wurde mit HPLC Sequenzanalyse kombiniert.
Miller CG und Bailey JM (August 1996), Automated C-Terminal Protein Sequence Analysis Using the HP G1009A C-terminal Protein Sequencing System, HP-Journal, Article 10*:* Eine Proteinprobe wird chemisch modifiziert, indem der C-terminale Aminosäurerest mit einem Kopplungsreagenz verknüpft wird. Danach wird die so markierte Probe proteolytisch gespalten. Die Spaltreaktion (als chemisches Spaltagens muss etwas eingesetzt werden, das mit dem Kopplungsreagens zusammenwirkt) produziert einen modifizierten C-terminalen Aminosäurerest i.e. Thiohydantoin-Aminosäure, der dann per HPLC analysiert werden kann. Die Aminosäurekette ist dann um eine Aminosäure verkürzt und ein neuer Reaktionszyklus wird gestartet.
US 6,046,053 beschreibt eine chemische Methode der C-terminalen Aminosäuresequenzierung, die in zwei Schritten erfolgt. Zuerst wird ein Protein oder Peptid mit einem Säureanhydrid zur Reaktion gebracht. Der N-Terminus wird dabei geschützt und der C-Terminus so modifiziert, dass ein Oxozolon entsteht. In weiterer Folge wird dieses Reaktionsprodukt mit einer Säure oder einem Alkohol zur Reaktion gebracht, um den C-terminalen Aminosäurerest freizusetzen. Diese Schritte werden wiederholt und sequenziell die freigesetzten C-terminalen Aminosäurereste isoliert und identifiziert. Alternativ wird die Verwendung eines halogenierten Ameisensäureesters statt Säureanhydrid im ersten Schritt und die Isolierung und Identifizierung der Aminosäure oder des Aminosäurederivats beschrieben.
Die im Stand der Technik verfügbaren Methoden zur C-terminalen Aminosäuresequenzierung verfolgen die Methodik, einzelne Aminosäuren vom C-Terminus der Sequenz her kommend abzuspalten und einzeln zu analysieren. Nachteil ist, dass nur sehr kurze Stücke der C-terminalen Aminosäuresequenz identifiziert werden können und eine hohe Fehlerrate besteht, die unter anderem mit der Komplexität des Proteins zusammenhängt. Keines der bislang beschriebenen Reaktionsschemata für die Routineanalyse der C-terminalen Aminosäurereste hat sich in der Praxis durchgesetzt.

Die Sequenz des C-Terminus einer Aminosäuresequenz zu kennen leistet einen wertvollen Beitrag zur Vervollständigung der strukturellen Integrität von nativen Proteinen, welche aus natürlichen Quellen isoliert oder nach genetischem Engineering rekombinant exprimiert werden. Da es bislang keine zuverlässig einsetzbaren Methoden zur Aufklärung der C-terminalen Aminosäuresequenz gibt, war Aufgabe der vorliegenden Erfindung, eine solche Methode zu etablieren. Eine weitere Aufgabe bestand darin, längere Stücke der C-terminalen Aminosäuresequenz zu identifizieren als dies im Stand der Technik beschrieben ist.

Zur Lösung der Aufgabe der C-terminalen Sequenzierung von Aminosäuresequenzen wurde ein Verfahren zur Sequenzierung des C-Terminus einer Aminosäuresequenz entwickelt, das dadurch gekennzeichnet ist, dass man:
a) die freien COOH-Gruppen im Protein mit Marker A umsetzt
b) freien Marker A entfernt
c) das markierte Protein in Peptide spaltet
d) die durch Schritt c neu freigewordenen COOH-Gruppen gegebenenfalls mit Marker B umsetzt, der sich von Marker A unterscheidet
e) das ausschließlich mit Marker A markierte C-terminale Peptidstück identifiziert und
f) das ausschließlich mit Marker A markierte C-terminale Peptidstück in üblicher Weise sequenziert.

Auf diese Weise lässt sich die Sequenz des C-Terminus einer Aminosäuresequenz durch die Anwendung der üblichen Methoden der Sequenzierung bestimmen. Die Methode ist eine deutliche Verbesserung gegenüber dem Stand der Technik, weil die Länge des C-terminalen Peptidfragmentes darüber entscheidet, wie viele Aminosäuren des C-Terminus sequenziert werden. Insgesamt vereint die erfindungsgemäße Methode die Vorteile der chemischen (selektive Markierung des C-Terminus, eventuell selektive chemische Spaltung des Proteins), physikalischen (Trennverfahren und Identifizierung des C-Terminus über einen Marker) und gegebenenfalls enzymatischen (ausreichend selektive Spaltung des Proteins) Strategien zur Identifizierung einer Aminosäuresequenz.
Gemeinsam mit der herkömmlich eruierten Sequenz des N-Terminus derselben Aminosäuresequenz, verfügt man z.B. über die wesentliche Ausgangsinformation für reverse Translation und damit die entsprechenden Nukleotidsequenzen (d.h. Oligonukleotide), die als solche als Primer verwendet werden können. Mittels Einsatz der Primer in der "Polymerase Chain Reaction" (PCR) kann die vollständige Nukleotidsequenz rekonstruiert und vervielfältigt werden.

Es werden nachfolgende Begriffe verwendet, die wie angegeben definiert sind:
"Aktivierung" von funktionellen COOH-Gruppen ist die über Umsetzung mit Reagenzien erreichte Erhöhung der Nukleophilie an dieser Position. Aktivierung wird auch als "Modifikation" bezeichnet.
"Aminosäuren" sind organische Verbindungen mit mindestens einer Carboxygruppe (-COOH) und einer Aminogruppe (-NH₂). Aminosäuren werden unterschiedlich eingeteilt:
   a) Isoelektrischer Punkt: sauer, basisch, neutral.
   b) Struktur der Seitenkette: aliphatisch, aromatisch, heterozyklisch. Spezialstrukturelemente: OH, S in der Seitenkette, oder Amin Bestandteil des Pyrrolidinringes.
   c) Polarität der Seitenkette: polar, unpolar.
   d) Struktur der beim Abbau des Kohlenstoffgerüstes gebildeten Verbindungen.
"Ausschlussgrenze" charakterisiert die stationäre Phase in der Gelchromatographie und wird durch den Vernetzungsgrad und die Porenweite der gequollenen Gele festgelegt. Größere Moleküle schlängeln sich durch die Zwischenräume der Säulenpackung hindurch, kleinere dringen in die Poren der Gelkörper ein und wandern deswegen langsamer als große. Die Ausschlussgrenze ist die minimale Molekülmasse eines globulären Moleküls, welches durch die stationäre Phase zurückgehalten wird.
"C-terminal" sind Sequenzen, die von der ursprünglichen vollständigen Proteinsequenz ausgehend, von einer alpha-Carboxygruppe begrenzt sind.

Die "DNA" (Desoxyribonukleinsäure) legt die Abfolge der Aminosäuren in einem Protein fest. Chemisch gesehen handelt es sich bei der DNA um eine Nukleinsäure, ein langes Kettenmolekül (Polymer) aus Einzelstücken, sogenannten "Nukleotiden", welche aus einem Phosphat-Rest, einem Zucker (i.e. Desoxyribose) und einer von vier organischen Basen Adenin (A), Guanin (G), Cytosin (C) und Thymin (T) bestehen. Die "RNA" ist auch eine Nukleinsäure, die sogenannte Ribonukleinsäure, bestehend aus einer Ribose (d.h. einer Pentose), einem Phosphatrest und einer organischen Base. Die Ribose der RNA ist mit derjenigen der DNA identisch, bis auf eine Hydroxylgruppe (statt eines Wasserstoff-Atoms) an der 2'-Position im Pentose-Ring (daher auch Desoxyribonucleinsäure DNA). In der RNA kommen die folgenden organischen Basen (auch als Nukleinbasen bezeichnet) vor: Adenin (A), Guanin (G), Cytosin (C) und Uracil (U).

"Elektromagnetische Strahlung" ist eine Welle aus gekoppelten elektrischen und magnetischen Feldern. Zu ihnen gehören unter anderem Radiowellen, Mikrowellen, Infrarotstrahlung, sichtbares Licht, UV-Strahlung sowie Röntgen- und Gammastrahlung - kurz, das gesamte elektromagnetische Wellenspektrum. Der einzige Unterschied zwischen diesen Wellentypen liegt in ihrer Frequenz und somit ihrer spezifischen Energie. Elektromagnetische Wellen verhalten sich immer auch wie Teilchen, man nennt sie "Photonen". Das für Menschen sichtbare "Licht" ist ein Bereich der elektromagnetischen Strahlung. Dieser erstreckt sich von etwa 380 bis 780 nm Wellenlänge. Die an das sichtbare Licht angrenzenden Bereiche der Infrarotstrahlung und Ultraviolettstrahlung werden häufig ebenfalls als Licht bezeichnet. Neben der wahrnehmbaren Farbe also der Wellenlänge oder der zugehörigen Frequenz wird Licht auch durch die Eigenschaften Kohärenz und Polarisation charakterisiert.

"Elution" ist das Herauslösen oder Verdrängen von adsorbierten Stoffen aus festen oder mit Flüssigkeit getränkten Adsorbentien (stationären Phasen) und Ionenaustauschern durch kontinuierliche Zugabe eines Lösungsmittels (Elutionsmittel = mobiler Phase). Die aus der Trennsäule fließende Lösung wird "Eluat" genannt.

"Farbstoffe" sind chemische Verbindungen, die die Eigenschaft haben, andere Materialien "farblich zu markieren". Die Farbigkeit beruht auf der Fähigkeit, aus dem Spektrum des sichtbaren Lichtes, das gemischt den Farbeindruck weiß ergibt, einen Teil zu absorbieren. Die Lichtabsorption besteht darin, dass Elektronen unter Aufnahme von Energie je eines Photons in einen höheren Energiezustand übergehen. Geeignete Molekülstrukturen heißen "Chromophore" und sind jene Grundstrukturen die delokalisierbare Elektronen enthalten. Die Art der Chromophore beeinflusst über ihre Absorption von Licht den Farbton des Farbstoffes. Chromophore werden üblicherweise über photometrische Methoden analysiert, die Messverfahren im Wellenlängenbereich des sichtbaren Lichtes darstellen.

"Identifizierung" umfasst die Abtrennung und Detektion des C-terminalen Peptidfragmentes, nicht aber die Sequenzierung des Peptidfragmentes.

"Lumineszenz" ist die elektromagnetische Strahlung eines physikalischen Systems, die beim Übergang von einem angeregten Zustand zum Grundzustand entsteht (strahlende Desaktivierung). Lumineszenz kann unter anderem auch nach der Dauer des "Leuchtens" nach Ende der Erregung eingeteilt werden:
o "Fluoreszenz" ist die kurzzeitige, spontane Emission von elektromagnetischer Strahlung bzw. Licht beim Übergang eines elektronisch angeregten Systems in einen Zustand niedrigerer Energie, wobei das emittierte Licht im Regelfall energieärmer ist als das vorher absorbierte. Bei dem Fluoreszenzanregungsspektrum handelt es sich um ein Spektrum, dessen Fluoreszenzemission bei einer konstanten Wellenlänge detektiert wird, während die Anregungswellenlänge verändert wird. Die Detektion erfolgt normalerweise beim Maximum der Fluoreszenzemission. Im Idealfall entspricht dabei das Fluoreszenzanregungsspektrum dem Absorptionsspektrum des "Fluorophors" (i.e. Verbindungen, die Photonen bei der Energie einer Wellenlänge absorbieren und die Energie bei einer anderen Wellenlänge emittieren), da die emittierte Strahlung proportional zur absorbierten Strahlung ist. Fluoreszierende Verbindungen werden als Farbstoffe (Fluoreszenzfarbstoffe) bezeichnet, weil wie die Chromophore auch die Fluorophore unter anderem durch den Parameter Wellenlänge von elektromagnetischer Strahlung charakterisiert sind. Alternativ wird von floureszenzmarkierten Stoffen oder Verbindungen gesprochen.
o "Phosphoreszenz" ist wie die Fluoreszenz eine besondere Form der Lumineszenz (kaltes Leuchten). Sie unterscheidet sich von der Fluoreszenz darin, dass die Fluoreszenz nach dem Ende der Bestrahlung rasch endet, meist innerhalb einer millionstel Sekunde, wogegen es bei der Phosphoreszenz zu einem Nachleuchten kommt, das von Sekundenbruchteilen bis hin zu Stunden dauern kann. Phosphoreszierende Stoffe werden auch als "Luminophore" bezeichnet, da sie das Licht scheinbar speichern.

"Marker" ist eine Verbindung, die an freie COOH-Gruppen eines Proteins oder Peptids binden können und das Protein oder Peptid kennzeichnet. Ein Marker kann an eine solche freie COOH-Gruppe direkt (z.B. durch nukleophile Substitution der Hydroxygruppe) oder nach Aktivierung derselben, aber jedenfalls gerichtet binden.

"Markierung" bedeutet Modifikation der funktionellen COOH-Gruppen im Protein bzw. den Peptidstücken (synonym verwendet wird Biokonjugation). "Differenzierte Markierung" liegt vor, wenn mindestens zwei voneinander unterscheidbare Marker vorhanden sind. "Differenziert" bezieht sich dabei auf die Unterscheidbarkeit der Marker z.B. aufgrund intrinsischer physikalischchemischer oder atomarer Eigenschaften der Marker, wie elektromagnetischer Strahlung z.B. in Form von Fluoreszenz, sichtbarerem farblichen (i.e. chromogenen) Charakter, oder Radioaktivität. Auch Bioaffinität mit anderen Strukturen kann eine solche für die Detektion nützliche Eigenschaft darstellen. Eine unterscheidbare Markierung liegt auch dann vor, wenn das schlichte Vorhandensein einer freien COOH-Gruppe, eine Unterscheidung von mit Marker gekennzeichneten anderen COOH-Positionen möglich ist.

"Mobile Phase" ist die Phase, in die ein Substanzgemisch am Beginn des Trennsystems eingebracht und die bewegt wird. Bei der Flüssigchromatographie ist die mobile Phase flüssig (auch "Eluent" genannt). Bei der Gaschromatographie kommen Trägergase wie Wasserstoff, Helium oder Stickstoff zum Einsatz, in der Dünnschichtchromatographie spricht man vom Fließmittel. Mobile Phasen unterscheiden sich in ihrer Elutionsfähigkeit ("Stärke", siehe dazu "Elutrope Reihe", die allgemeines Fachwissen darstellt), dies bedingt unterschiedliche Retentionszeiten und oft auch unterschiedliche Selektivitäten.

"N-terminal" sind Sequenzen, die von der ursprünglichen vollständigen Proteinsequenz ausgehend, von einer alpha-Aminogruppe begrenzt sind.

"Nukleophilie" ist ein Maß für die Fähigkeit eines negativ polarisierten Moleküls ein positiv polarisiertes oder geladenes Atom in einem Molekül unter Ausbildung einer kovalenten Bindung anzugreifen. Typische Nukleophile sind oft negativ geladen, oder haben eine stark negative Partialladung.

"Nukleotidsequenz" bezeichnet das Kettenmolekül der DNA bzw. RNA, bestehend jeweils aus einer Abfolge von Nukleotiden.

"Primer" ist ein Oligonukleotid, das als Startpunkt für DNA-replizierende Enzyme wie die DNA-Polymerase dient. DNA-Polymerasen benötigen eine Hydroxylgruppe als Startpunkt für ihre erste Verknüpfungsreaktion. Primer stellen mit ihrem 3' OH-Ende eine passende Hydroxylfunktion zur Verfügung. Bei der In-vitro-Amplifikation von DNA, beispielsweise bei der PCR, werden Primer benötigt, um den spezifischen DNA-Abschnitt, der amplifiziert werden soll, zu flankieren und damit festzulegen. Ein Primer repräsentiert hierbei jeweils den gegenläufigen Strang zu seinem "Primerpartner", i.e. der Nukleotidsequenz der Probe. Primer für PCR-Ansätze haben in der Regel eine Länge von 18-30 Nukleotiden.

"Proteine", "Polypeptide" und "Oligopeptide" sind Ketten aus Aminosäuren, die sich in ihrer Länge und ihrer Abfolge (i.e. Aminosäuresequenz) unterscheiden.

"Protein(bio)synthese" oder "Genexpression" ist die Herstellung eines Proteins bzw. Polypeptids. In einem ersten Schritt der Protein(bio)synthese wird ein Gen aus der DNA abgelesen und in ein mRNA (messenger-RNA)-Molekül "transkribiert": Die "Transkription" des Gens wird durch das Enzym RNA-Polymerase (und mehrere andere Proteine) bewerkstelligt, das als Substrat DNA und die Ribonukleosidtriphosphate ATP, UTP, CTP und GTP benötigt. Daraus wird komplementär zu einem DNA-Strang eine fortlaufende RNA-Kette (mRNA) unter Abspaltung jeweils zweier Phosphatreste der Triphosphate hergestellt. Bei diesem Vorgang werden die Nukleinbasen der DNA (A, T, G, C) in die Nukleinbasen der RNA (A, U, G, C) umgeschrieben. Anstelle des T kommt U und anstelle der Desoxyribose kommt Ribose in der RNA vor. Unter "Translation" versteht man die Übersetzung der Basensequenz der mRNA in die Aminosäuresequenz des Proteins. In der mRNA bilden drei aufeinander folgende Basen ein Codon (auch Basentriplett), welches für eine Aminosäure codiert. Die Aminosäuren werden entsprechend der Abfolge der Codons sequentiell translatiert.

"Proteinsequenz" und "Aminosäuresequenz" werden synonym verwendet. Die Abfolge der einzelnen Aminosäuren wird im Allgemeinen als Primärstruktur eines Proteins bezeichnet, wobei die einzelnen Aminosäuren amidartig miteinander verbunden sind, die sich "Peptidbindung" nennt. Gespaltene Aminosäuresequenzen werden als "Peptidsequenzen" oder "Peptidfragmente" bezeichnet, wobei "Spaltung" die Auflösung einer Peptidbindung, d.h. der Amidbindung bedeutet.

"Retention" ist die Verzögerung von einzelnen Substanzen des Substanzgemisches durch Wechselwirkung mit der stationären Phase (die Zeit, die die einzelnen Substanzen individuell für die Durchwanderung der stationären Phase benötigt wird, heißt Retentionszeit). Die Stärke der Wechselwirkungen zwischen den Komponenten eines Substanzgemisches und der stationären Phase wird sowohl von deren Struktur als auch von deren funktionellen Gruppen bestimmt. Dabei treten bei unpolaren Substanzen ausschließlich Dispersionswechselwirkungen (Van-der-Waals-Bindung) auf, während polare Trennphasen auch polare Wechselwirkungen eingehen können, etwa Wasserstoffbrückenbindungen oder Donator-Akzeptor-Bindungen.

"Reversed Phase" (RP) oder "Umkehrphase" ist eine Möglichkeit bei der Adsorptionschromatographie, ein Substanzgemisch zu trennen, i.e. unpolare stationäre Phase (wie modifiziertes Kieselgel) und polare mobile Phase (wie gepuffertes Wasser). Im Gegensatz dazu wird unter normaler Phase der Einsatz von einer polaren stationären Phase (wie z.B. Kieselgel, Aluminiumoxid), und unpolaren bis mittelpolaren mobilen Phasen (wie z.B. Kohlenwasserstoffe, Dioxan, Essigsäureethylester) verstanden. In der HPLC wird oft RP und eine Gradienteneluierung angewandt, wobei die Zusammensetzung des Lösungsmittels langsam geändert wird (z.B. von 80 % auf 20 % Wasseranteil).

"Reverse Translation" heißt die Generierung von einer DNA Sequenz auf Basis einer bekannten Proteinsequenz. Diese Technik wird vor allem dort verwendet, wo Primer für eine PCR gebraucht werden.

"Selektivität" drückt aus, inwieweit die Bestimmung einer Komponente in einem Stoffgemisch durch andere Komponenten gestört wird. Eine Analysenmethode zeichnet sich dann als qualitativ hochwertig aus, wenn sie selektiv ist, d.h. für die Einzelkomponente spezifisch ist.

"Stationäre Phase" (auch Trennbett genannt) ist die Phase in einem chromatographischen System, die mit den einzelnen Substanzen eines Substanzgemisches Wechselwirkungen eingeht und sich nicht bewegt. Der Aufenthalt der einzelnen Substanzen bei ihrer Retention wechselt zwischen mobiler und stationärer Phase (random walk), und verursacht die substanzcharakteristische Retentionszeit. Stationäre Phase können z.B. Flüssigkeiten, Gele, oder Feststoffe sein.

Nachfolgend wird die Erfindung eingehend beschrieben:
Aminosäuren sind Verbindungen mit mindestens einer Carboxygruppe (-COOH) und einer Aminogruppe (-NH₂). NH₂ als funktionelle Gruppe ist basisch während COOH als funktionelle Gruppe aufgrund der Elektronegativitätsdifferenz zwischen dem Sauerstoff und Kohlenstoff am Kohlenstoffatom eine positive Partialladung trägt. COOH ist daher als funktionelle Gruppe weniger nukleophil als NH₂.
Das erfindungsgemäße Verfahren basiert auf der eindeutigen Identifizierung der C-terminalen Sequenz eines Proteins nach selektiver Spaltung in Peptidstücke, die verschieden ist von allen anderen so entstandenen Peptidstücken. Dies erfolgt beispielsweise über differenzierte Markierung dieser Peptidstücke. Markierung bedeutet erfindungsgemäß die Anbringung eines Markers an die freien funktionellen COOH-Gruppen im Protein bzw. den Peptidstücken.
Die Verfahrensschritte:

### Schritt a: Markierung der COOH-Gruppen des Proteins mit Marker A

Die Markierung erfolgt über Verbindungen (z.B. geeignete Hydrazine, Hydroxylamine oder Amine), welche eine empfindliche Detektion ermöglichen z.B. durch intrinsische physikalischchemische oder atomare Eigenschaften, wie elektromagnetischer Strahlung z.B. in Form von Fluoreszenz, sichtbarerem farblichen (i.e. chromogenen) Charakter, oder Radioaktivität. Auch Bioaffinität mit anderen Strukturen kann eine solche für die Detektion nützliche Eigenschaft darstellen. Verbindungen, die in Schritt a zur Markierung verwendet werden können, sind hinlänglich bekannt und die Markierung erfolgt nach bestehenden Protokollen (siehe z.B. Hermanson (1996), Bioconjugate Techniques, Academic Press, Kapitel 8, beginnend auf Seite 297). Bindet ein Marker an die reaktive Gruppe eines Proteins (Ziel ist hier die gegebenenfalls aktivierte COOH-Gruppe), so ist das Protein oder Peptid mit einer Markierung versehen, die detektiert und damit lokalisiert werden kann. Die Natur des verwendeten Markers entscheidet über das zu verwendende Detektionssystem. Die Detektion erfolgt üblicherweise über drei generelle Prinzipien (siehe dazu z.B. Hermanson (1996), Bioconjugate Techniques, Academic Press, Kapitel 8, beginnend auf Seite 297): spektro-photometrische oder radiosensitive Instrumente, oder indirekt über andere markierte Verbindungen. Erfindungsgemäß bevorzugt sind spektro-photometrisch detektierbare Verbindungen (hier Farbstoffe genannt), insbesondere solche die chromogen oder lumineszierend sind. Besonders bevorzugt sind Fluoreszenzfarbstoffe, welche z.B. Hydrazine, Hydroxylamine oder Amine sind. Die Mehrheit der Fluorophore für die Markierung von Biomolekülen wie Proteinen, enthält ein aromatisches Ringsystem, das für die Lumineszenz verantwortlich ist. Die Bestandteile des aromatischen Ringsystems wiederum beeinflussen die Fluoreszenz, wobei die Quantenausbeute Q (i.e. Verhältnis der Photonenemission zur-absorption) möglichst hoch sein soll. Die Qualität des detektierten Signals ist umso besser, je größer der Stoke's shift (Trennung der Wellenlängen von Absorptions- und Emissionsmaximum) ist. Beispiele für Fluoreszenzfarbstoffe sind (siehe dazu Hermanson (1996), Bioconjugate Techniques, Academic Press, Kapitel 8, Abschnitt 1, beginnend auf Seite 298; und Haugland RP (2002), Handbook of Fluorescent Probes and Reserach Products, Molecular Probes, Kapitel 3, Abschnitt 3.3, beginnend auf Seite 112 und Molecular Probes Manual, Kapitel 3.3, http://www.invitrogen.com/site/us/en/home/References/Molecular-Probes-The-Handbook.html):
o Fluoresceine, wie z.B. Fluorescein Cadaverin, Alexa Fluor® 488 Cadaverin
o Rhodamine, wie z.B. Texas Red® Cadaverin
o Cumarin Derivate mit der Basisstruktur 2H-1-Benzopyran-2-on, z.B. Alexa Fluor® 350 Cadaverin
o BODIPY® Farbstoffe haben 4,4-Difluoro-4-bora-3a, 4a-diaza-s-Indazen als Grundgerüst. Beispiele: BODIPY® FL Ethylendiamin, BODIPY® TR Cadaverin
o Cascade Blue® Derviate, deren Basisstruktur Trisulfonierte Pyrene sind, wie z.B. Cascade Blue® Ethylendiamin, Cascade Blue® Cadaverin
o Lucifer Yellow® Farbstoffe sind 3,6-Disulfonat 4-Aminonaphthalimide. Beispiele: Lucifer Yellow® Ethylendiamin, Lucifer Yellow® Cadaverin

Um eine Reaktion der COOH-Gruppen mit den gängigen Markern (Biokonjugation erfolgt meist über nukleophile Addition), welche bevorzugt Fluoreszenzfarbstoffe sind (siehe dazu Haugland RP (2002), Handbook of Fluorescent Probes and Reserach Products, Molecular Probes, Kapitel 3, Abschnitt 3.3, beginnend auf Seite 112 und Molecular Probes Manual, Kapitel 3.3, http://www.invitrogen.com/site/us/en/home/References/Molecular-Probes-The-Handbook.html), zu erleichtern, werden diese funktionellen COOH-Gruppen bevorzugt aktiviert, indem andere Gruppen an COOH binden, die nukleophiler verglichen mit NH₂ sind, d.h. die COOH-Gruppen modifizieren. Es ist eine Reihe solcher Reaktionen bekannt (siehe dazu beispielsweise Hermanson (1996), Bioconjugate Techniques, Academic Press, Seite 152-154 und Kapitel 3, Abschnitt 1, beginnend auf Seite 169). Beispielsweise können reaktive Ester mit Diazoacetatderivaten gebildet werden, oder mit Carbonyldiimidazolen reaktive Imodazolderivate, oder mit Carbodiimiden Amin-enthaltende Verbindungen generiert werden.
Die Verwendung von 1-Ethyl-3-(3-dimethyl-aminopropyl)-carbodiimid-hydrochlorid (EDC) ist die bekannteste Methode zur Modifikation von COOH-Gruppen in wässriger Lösung. In diesem Fall findet die optimale Reaktion bei pH Werten zwischen 4 und 6 statt (Hydrolyserate der Ester ist geringer bei sauren pH-Werten). Die Reaktion kann aber auch bei pH Werten von 6 bis 9 effizient ablaufen.
Das erfindungsgemäße Verfahren wird vorzugsweise in wässrigem Medium durchgeführt, weswegen eine etwaige Aktivierungsreaktion vorzugweise unter Verwendung wasserlöslicher Aktivierungsreagenzien im wässrigen Medium stattfindet. Der pH wird im Allgemeinen durch Zugabe von Säuren wie z.B. HCl konstant gehalten. Der Einsatz von gepufferten Lösungen ist vorteilhaft. Die Wahl des pH Wertes des Lösemittels richtet sich nach der Wahl des nachfolgend zu verwendenden Markers. Bei niedrigen pH Werten sind beispielsweise Fluoresceinylglycinamid (5-(Aminoacetamido) fluorescein) und verschiedene Hydrazine und Hydroxylamine reaktiver als aliphatische Amine wie z.B. Cadaverine. (Vorschrift zur Durchführung siehe Hermanson (1996), Bioconjugate Techniques, Academic Press, Seite 170-173). Im Allgemeinen sind für Markierungen Lösemittel im neutralen oder schwach basischen pH Bereich bevorzugt, i.e zwischen 7 und 9.
Die Aktivierung kann im Speziellen mit EDC und N-Hydroxysulfosuccinimid (Sulfo-NHS, wasserlöslich) erfolgen (die Reaktion erfolgt über bekannte Vorschriften; siehe dazu z.B. Hermanson (1996), Bioconjugate Techniques, Academic Press, Seite 173-176): EDC reagiert mit einer Carboxygruppe, und ein Amin-reaktives O-acyl-isoharnstoff-Intermediat entsteht. Dieses Intermediat kann mit der Amingruppe eines zweiten Moleküls reagieren, sodass ein Konjugat von zweien mit einer Amid-Bindung verbundenen Molekülen entsteht. Um die Stabilität im wässrigen Medium zu erhöhen, kann es vorteilhaft sein, durch Zugabe von Sulfo-NHS das Amin-reaktive Intermediat in einen Amin-reaktiven Sulfo-NHS-Ester umzuwandeln.
1-Cyclohexyl-3-(2-morpholinoethyl)carbodiimid (CMC) ist neben EDC das gängigste Carbodiimid für COOH-Modifikationen in wässriger Lösung (siehe dazu Hermanson (1996), Bioconjugate Techniques, Academic Press, Seite 177).

COOH-Modifikationen können aber nicht nur in wässrigem Medium, sondern auch in organischer Lösung durchgeführt werden, was den Vorteil hat, dass Hydrolyse nicht auftritt. Eine solche Methode ist beispielsweise die Umwandlung von COOH-Gruppen zu Succinimidylestern oder gemischten Anhydriden. Dicyclohexylcarbodiimid (DCC, siehe dazu Hermanson (1996), Bioconjugate Techniques, Academic Press, Seite 177-180) und Diisopropylcarbodiimid (DIC, siehe dazu Hermanson (1996), Bioconjugate Techniques, Academic Press, Seite 180-181) werden häufig eingesetzt, um die Amidbildung in organischen Lösungsmitteln wie beispielsweise DMF (N,N-Dimethylformamid), DMSO (Dimethylsulfoxid), oder THF (Tetrahydrofuran) zu ermöglichen. Eine weitere Methode zur Kopplung von COOH-Verbindungen in organischen Lösungsmitteln an aliphatische Amine ist die Kombination von 2,2'-Dipyridyldisulfid und Triphenylphosphin (siehe dazu Haugland RP (2002), Handbook of Fluorescent Probes and Reserach Products, Molecular Probes, Kapitel 3, Abschnitt 3.3, beginnend auf Seite 112 und Molecular Probes Manual, Kapitel 3.3, http://www.invitrogen.com/site/us/en/home/References/Molecular-Probes-The-Handbook.html).
COOH-Gruppen können auch mit Woodward's Reagent K (N-ethyl-3-phenylisoxazolium-3'-sulfonat) oder N,N'-Carbodiimidazole (CDI) modifiziert werden, um eine Weiterreaktion mit Markern zu begünstigen (siehe dazu Hermanson (1996), Bioconjugate Techniques, Academic Press, Seite 181-185).
Alternativ zum Einsatz von Reagenzien zur Aktivierung der COOH-Gruppen und nachfolgender Markierung kann auch die Modifikation des Proteins (Schritt a) bzw. der Peptidfragmente (Schritt d) durch Verwendung eines bereits z.B. mit fluoreszenzmarkierten Carbodiimides erfolgen wie z.B. N-cyclohexyl-N'-(4-(dimethylamino)naphthyl)carbodiimide (auch NCD-4 genannt, siehe dazu beispielsweise Haugland RP (2002), Handbook of Fluorescent Probes and Reserach Products, Molecular Probes, Kapitel 3, Abschnitt 3.3, beginnend auf Seite 112 und Molecular Probes Manual, Kapitel 3.3,
http://www.invitrogen.com/site/us/en/home/References/Molecular-Probes-The-Handbook.html).

### Schritt b: Abtrennung von freiem Marker A

Die Abtrennung des etwaig freien Markers A von der in Schritt a markierten Proteinprobe verhindert die Bindung von Marker A an in weiterer Folge frei werdende COOH-Gruppen. Eine solche Abtrennung erfolgt über allgemeine Trennprinzipien für die Proteinaufreinigung, die die physikalisch-chemischen Eigenschaften der Proteine bzw. Peptide nutzt (siehe beispielsweise Sewald N, Jakubke HD (2009) Peptides: Chemistry and Biology, Wiley-VCH, Kapitel 2.3.1, beginnend auf Seite 13. Jakubke HD und Jeschkeit H (1982) Aminosäuren, Peptide, Proteine, Akademie-Verlag, Kapitel 3.3, beginnend auf Seite 388):
o "Löslichkeit" beispielsweise genutzt in Fällungsreaktionen (isoelektrische Fällung, Lösungsmittelfällung, Neutralsalzfällung), Multiplikativer Verteilung nach Craig, und Verteilungschromatographie
o "Molekülgröße" beispielsweise genutzt in Dialyse, Ultrafiltration, und Gelchromatographie (Gelfiltration, Gelpermeationschromatographie)
o "Molekülgestalt" beispielsweise genutzt in Zentrifugationsverfahren
o "Elektrische Ladung" beispielsweise genutzt in Elektrophoreseverfahren (trägerfrei oder mit Träger wie Gelelektrophorese und isoelektrische Fokussierung) und lonenaustauschchromatographie
o "Protein-Ligand-Wechselwirkung" beispielsweise genutzt in Affinitätschromatographie

Die Trennverfahren erfolgen nach bekannten Vorschriften und sind dem Fachmann bekannt, ebenso die Parameter, die dem ausgewählten Trennverfahren innewohnen. Ziel ist eine selektive Trennung der gewünschten Einzelkomponente vom Rest, hier die Trennung des markierten Proteins von freiem Marker A.
Bevorzugt erfolgt eine Abtrennung aufgrund der unterschiedlichen Größe der vorliegenden Bestandteile, i.e. freier Marker und markiertes Protein. Besonders bevorzugt ist die Auftrennung aufgrund der Molekülgröße in einer Säule, bei der alle Komponenten den gleichen Weg durch ein definiertes Trennbett (i.e. stationäre Phase) zurücklegen, aber aufgrund unterschiedlicher Wechselwirkungen mit der stationären Phase zu unterschiedlichen Zeiten die Säule wieder verlassen und damit zeitlich versetzt detektiert werden können. Diese, auch Größenausschlusschromatographie (SEC, size exclusion chromatography; synonym mit Gelfiltration) genannte Methode, ist eine besondere Form der Flüssigchromatographie. Sie beruht auf der Trennung von Molekülen aufgrund ihrer unterschiedlichen Größe, d.h. an Kügelchen aus beispielsweise Kieselgel, Polymer, oder vernetzten Polysacchariden. Bevorzugt sind vernetzte Gele auf Polysaccharid- (z.B. alkylierte Dextrane vernetzt mit Epichlorhydrin) und Polyacrylamidbasis. Je nach Vernetzungsgrad und Porengröße, die die Ausschlussgrenze bestimmen, der in gequollenem Zustand vorliegenden Gele wandern Moleküle in Abhängigkeit von ihrer Größe (und Gestalt) mit unterschiedlichen Geschwindigkeiten durch die Säule. Die Wahl der mobilen Phase richtet sich nach der Art der stationären Phase:
o Werden hydrophile Säulenpackungen verwendet, so werden als mobile Phase wässrige Eluenten verwendet, die in der Regel Puffer zur pH Kontrolle wie beispielsweise Phosphatpuffer enthalten. Alternative Puffersysteme und ihre Pufferkapazitäten sind dem Fachmann bekannt.
o Bei hydrophoben Füllmaterialien werden entsprechend unpolare organische Solventien eingesetzt, wobei dieses Verfahren nur bei gering wasserlöslichen Substraten Verwendung findet. Siehe dazu Matthias Otto (1995), Analytische Chemie, VCH, Kapitel 5.3.3, beginnend auf Seite 480.

Eine effektive Trennung in chromatographischen Systemen wird einerseits bestimmt durch den Trennfaktor (Verhältnis Verteilungskoeffizient bzw. der Kapazitätsfaktoren), welcher die Selektivität des Phasensystems beschreibt. Variieren kann man den Trennfaktor durch die Wahl der Temperatur in der Gaschromatographie bzw. die Zusammensetzung der mobilen Phase in der Flüssigchromatographie. Es kann aber auch die stationäre Phase gewechselt werden. Die chromatographische Auflösung andererseits bezieht noch weitere Faktoren ein, die die Gesamtselektivität der Trennung beschreiben. Die Trennstufenanzahl, die sich aus der Trennstufenhöhe (i.e. Verhältnis der Varianz des Peaks und der Wanderungsgeschwindigkeit) bestimmt, ist von Bedeutung und kann z.B. durch die Säulenlänge variiert werden. Die Trennstufenhöhe kann durch die Veränderung der Strömungsgeschwindigkeit, der Korngröße der Packung, der Viskosität der Phasen und damit der Diffusionskoeffizient und/oder durch die Dicke des Films einer immobilisierten Flüssigkeit als stationäre Phase variiert werden. Die Auswahlkriterien für die einzelnen Chromatographieformen und die zugehörigen Verfahrensparameter sind dem Fachmann bekannt und sind beispielsweise nachzulesen in Matthias Otto (1995), Analytische Chemie, VCH, Kapitel 5, beginnend auf Seite 413.

### Schritt c: Spaltung von Peptidbindungen

Ziel ist es, ein C-terminales Peptidfragment herzustellen. Die Spaltung des markierten Proteins in Peptidfragmente erfolgt beispielsweise durch chemische und/oder enzymatische Partialhydrolyse:
Bei der enzymatischen Spaltung von Proteinen werden sogenannte (Carboxy-) Peptidasen eingesetzt. Exopeptidasen können in Aminopeptidasen, Carboxypeptidasen sowie Di- und Tripeptidasen unterteilt werden. Am C-Terminus agierende Exopeptidasen setzen einzelne Aminosäuren durch die Wirkung von Carboxypeptidasen oder Dipeptide durch die von Peptidyl-Dipeptidasen katalysierten Reaktionen frei.
Vorzugsweise werden erfindungsgemäß Endopeptidasen zur enzymatischen Peptidspaltung verwendet, welche innerhalb des Proteins Peptidbindungen möglichst spezifisch angreifen (siehe dazu Sewald N, Jakubke HD (2009) Peptides: Chemistry and Biology, Wiley-VCH, Seite 26). Zu den Endopeptidasen gehören beispielsweise die nachfolgend genannten:
o Trypsin ist eine Serinprotease und spaltet selektiv Peptidbindungen nach den Aminosäuren Lysin (Lys) und Arginin (Arg) - C-terminale Lys oder Arg Reste entstehen. Die Selektivität der Spaltung kann durch Blockierung der Seitenkettenfunktionen des Lysins (e-Aminogruppe; mit Acylierungsagens; wird 2-methylmaleic anhydride verwendet, so kann unter mild sauren Bedingungen auch wieder deblockiert werden) oder des Arginins (Cyclohexanon-Derivat der Guanido-Gruppe, siehe dazu Toi K, Bynum E, Norris E, Itano HA (1967). J. Biol. Chem. 242, Seite 1063) weiter erhöht werden. Cystein (Cys) kann einer Spaltung durch Trypsin zugänglich gemacht werden (nach Aminoalkylierung durch Haloalkylamin).
o Thrombin ist spezifischer als Trypsin, kann aber nur eine begrenzte Anzahl an Arg Bindungen spalten.
o Clostripain (aus Clostridium histolyticum) kann selektiv Arg Bindungen hydrolysieren. Lys Bindungen werden zu einem geringeren Anteil gespalten.
o V8 (aus Staphylococcus aureus Stamm V8) spaltet Peptidbindungen an der carboxyischen Seite von Glutaminsäure (Glu).
o Chymotrypsin spaltet Peptidbindungen so, dass aromatische und hydrophobe Aminosäuren am C-Terminus entstehen.
o Papain ist wenig spezifisch, greift aber hauptsächlich Arg, Lys und Glycin-Reste (Gly) an. Nicht gespalten werden saure Aminosäuren.
o Subtilisin spaltet v.a. Peptidbindungen in Nachbarschaft von Serin (Ser), Gly und aromatischen Aminosäuren.
o Pepsin besitzt wenig ausgeprägte Seitenkettenspezifität. Gespalten werden Tryptophan-(Trp), Phenylalanin- (Phe), Tyrosin- (Tyr), Methionin- (Met) und Leucinbindungen (Leu).
o Elastase ist weniger spezifisch und hydrolysiert v.a. die Bindungen von neutralen Aminosäuren.
o Thermolysin (aus Bacillus thermoproteolyticus) spaltet vorwiegend nach Aminosäureresten mit hydrophober Seitenkette.

Im Allgemeinen verläuft die enzymatische Hydrolyse umso spezifischer, je kürzer die Reaktionszeiten gewählt werden. Dabei ist die Reinheit der verwendeten Enzyme von Bedeutung. Verfahren in Lösung werden häufig durch die Inaktivierung des eingesetzten Enzyms gekennzeichnet, um eine Beschränkung der Reaktionszeit zwischen Enzym und Proteinprobe zu gewährleisten. Bevorzugt sind Spaltsysteme bei denen Enzyme auf einer stationären Phase aufgebracht sind wie beispielsweise inerten, porösen Materialien wie Agarose, Glas Beads, Polyacrylamid, oder quervernetzten Dextranen (siehe dazu Sewald N, Jakubke HD (2009) Peptides: Chemistry and Biology, Wiley-VCH, Seite 15), da die Proteinprobe auf diese Weise einer definierten Reaktionszeit mit dem Enzym ausgesetzt ist.
Neben den angeführten enzymatischen Methoden sind noch spezifische chemische Verfahren zur Spaltung von Peptidketten in Gebrauch. So spaltet beispielsweise Bromcyan (siehe dazu Gross E, Witkop B. (1961); J. Am Chem. Soc. 83, Seite 1510) Peptidbindungen mit Beteiligung der Carboxygruppen von Met (unter Ausbildung von Peptidylhomoserinlakton und Freisetzung von Aminoacylpeptid) und N-Bromsuccinimid (NBS, siehe dazu Patchornik A, Lawson WB, Witkop B (1958); J. Am Chem. Soc. 80, Seite 4747. Mahoney WC, Smith PK, Hermodson MA (1981); Biochemistry 20, Seite 443.) solche mit Bindungen des Tyr und Trp.

### Schritt d: Gegebenenfalls Markierung der freigewordenen COOH-Gruppen der Peptidfragmente mit B

Wesentlich in diesem Schritt ist die Gewährleistung, dass ein Trennsystem für die entstandenen Peptidfragmente zur Verfügung steht, das in Schritt e die Identifikation des ausschließlich mit Marker A markierten C-terminalen Peptidfragmentes erlaubt (siehe dazu Beschreibung von Trennprinzipien in Schritt b).
An dieser Stelle sei nochmals darauf hingewiesen, dass der Marker B sich von Marker A physikalisch-chemisch unterscheidet. In einer besonderen Ausführungsform dienen die freien COOH-Gruppen selbst zur Unterscheidung von mit Marker A gekennzeichneten COOH-Gruppen.
Über die Verwendung eines eine Markierung enthaltenden Reagenzes zur Aktivierung der COOH-Gruppen besteht eine Detektionsmöglichkeit, über das das C-terminale Peptidfragment von den übrigen Peptidfragmenten unterschieden werden kann. Beispielsweise können die neu frei gewordenen COOH-Gruppen mit EDC und Sulfo-NHS-Biotin zur Reaktion gebracht werden, oder mit einem floureszenzmarkierten Carbodiimid (siehe Beschreibung der Aktivierung in Schritt a).
Alternativ kann eine weitere Markierung der frei gewordenen COOH-Gruppen an den Peptidfragmenten mit einem von A verschiedenen Marker beispielsweise einem weiteren Fluoreszenzfarbstoff erfolgen, dessen Fluoreszenzwellenlänge sich vom Marker A unterscheidet. Hier gelten dieselben Kriterien für die Markierung wie in Schritt a beschrieben. Für die weiteren Schritte kann es dann vorteilhaft sein, wie in Schritt b gezeigt, auch freien Marker B abzutrennen. Dies ist abhängig von der nachfolgend gewählten Identifizierungsmethode der Peptidfragmente.
Die Entscheidung über die gegebenenfalls verwendete Markierung erfolgt über die zur Verfügung stehenden Verfahren zur Unterscheidung bzw. Trennung zwischen Marker A und Marker B (siehe Schritt e). Die allgemeinen Trennverfahren sind unter Schritt b beschrieben.

### Schritt e: Identifizierung des mit ausschließlich A markierten Peptids

"Identifizierung" ist in der vorliegenden Schrift das eindeutige Erkennen des C-terminales Peptidstückes, das durch die vorangegangenen Schritte entstanden ist. Identifizierung umfasst die Abtrennung und Detektion des C-terminales Peptidfragmentes. Ziel ist die selektive Trennung des gewünschten C-terminalen Peptidfragmentes von den anderen Peptidfragmenten. Identifizierung ist nicht die anschließend in Schritt f zu erfolgende Analyse der Aminosäuresequenz des C-terminalen Peptidfragmentes.
Die Identifizierung ist abhängig von den physikalischen Eigenschaften der gewählten Markierungen und erfolgt zuerst über eine Trennung (siehe dazu allgemeinen Trennverfahren unter Schritt b) zwischen mit A und gegebenenfalls mit A+B markierten Fragmenten beispielsweise über chromatographische Methoden wie der HPLC (Hochleistungsflüssigkeitschromatographie). Besonders bevorzugt zur Identifizierung von Peptidfragmenten ist die Umkehrphasen HPLC (reversed phase, RP). Verwendet werden vorteilhaft mit üblichen Trägermaterialien gepackte Säulen. Dies kann eine vollständig hydrolysierte Kieselgeloberfläche z.B. mit Silanol-(Hydroxyl)-Gruppen sein, welche hydrophobisiert werden. Dies wird häufig über die Bindung von Alkylsilanen bewerkstelligt, wobei als Alkylreste z.B. C₄ bis C₁₈ Reste und besonders vorteilhaft C₁₂ Reste dienen. Es ist bekannt, dass die Retentionszeit zunimmt, je länger der Alkylrest ist. Solche Säulen sind in den üblichen polaren mobilen Phasen aus Wasser, Methanol oder Acetonitril in großen pH Bereichen (ca. 1,5-10) stabil. Entweder muss das Trennbett der Säule nach dem Lösemittel ausgewählt werden, in dem gearbeitet wird, oder umgekehrt. Die Bedingungen sind so zu wählen, dass die Stabilität der Säule während des Trennprozesses aufrechterhalten ist. Dem Fachmann sind die zu berücksichtigenden Kriterien bekannt.
Im Falle, dass kein Marker B angebracht worden ist, kann die Abtrennung beispielsweise über eine Affinitätssäule mit immobilisiertem Carbodiimid erfolgen. Auf eine geeignete Matrix, i.e. stationäre Phase wie beispielsweise Agarose, Polyacrylamid, oder Trägermaterialien, die mit geeigneten Kopplungsverfahren COOH-reaktive Reagenzien wie beispielsweise die zur Aktivierung von COOH-Gruppen beschriebenen Stoffe (siehe Schritt a) binden. Reagenzien und Verfahren sind in der Literatur ausführlich beschrieben (Jansson, JC und Rydén, L (1998), Protein Purification, John Wiley & Sons Verlag, Seiten 375-442) und dem Fachmann bekannt.
Im Falle der Verwendung eines eine Markierung enthaltenden Reagenzes zur Aktivierung der COOH-Gruppen z.B. nach Reaktion mit EDC und Sulfo-NHS-Biotin ist eine Abtrennung der Peptidfragmente vom nur mit Marker A versehenen C-terminalen Peptidfragment über z.B. eine Streptavidin-Agarose Affinitätssäule möglich.
Die anschließende Detektion und damit Unterscheidung der Peptidfragmente wird durch die Natur der verwendeten Marker determiniert und erfolgt über übliche Verfahren (wie in Schritt a und beispielsweise in Hermanson (1996), Bioconjugate Techniques, Academic Press, Kapitel 8, beginnend auf Seite 297 beschrieben) und kann z.B. über einen UV und/oder Fluoreszenzdetektor geschehen.

### Schritt f: Sequenzierung der C-terminalen Sequenz

Die Aminosäuresequenz des C-terminalen Peptidfragmentes wird über übliche Verfahren bestimmt. Eine gängige Methode ist die N-terminale Sequenzierung gemäß dem von Edman etablierten Prinzip (siehe dazu beispielsweise Sewald N, Jakubke HD (2009) Peptides: Chemistry and Biology, Wiley-VCH, Seite 28-30).
Eine andere Ausführungsform des erfindungsgemäßen Verfahrens bevorzugt die Massenspektrometrie (siehe dazu Sewald N, Jakubke HD (2009) Peptides: Chemistry and Biology, Wiley-VCH, Seite 31). Biomoleküle wie Proteine und Peptide werden dabei ionisiert (z.B. durch MALDI oder ESI). Diese Methode stellt eine echte Alternative zum Edman-Abbau dar. Im Speziellen können kurze Peptidfragmente wie ein C-terminales Bruchstück direkt sequenziert werden können.
Eine Kombination von Edman Abbau und Massenspektrometrie stellt das Peptide Ladder Sequencing dar (siehe dazu Sewald N, Jakubke HD (2009) Peptides: Chemistry and Biology, Wiley-VCH, Seite 32-33)

Im folgenden wird der Versuchsablauf an einem praktischen Beispiel wiederum in den vorgenommen Verfahrensschritten abgehandelt. Alle Beispiele wurden mit dem Testprotein β-Lactoglobulin A durchgeführt. Als Puffer, Lauf- und Lösemittel wurde ein gängiger Phosphatpuffer verwendet. Das Beispielverfahren wurde bei Raumtemperatur durchgeführt.

### Beispiel für Schritt a: Kopplung freier COOH-Gruppen mit Marker A

Als Marker A wurde ein Fluoreszenzfarbstoff verwendet, i.e Alexa Fluor 488 Cadaverine. Die Markierung erfolgte nach Aktivierung der freien COOH-Gruppen mit EDC/Sulfo-NHS. Verwendete Materialien:
o Protein: β-Lactoglobulin A aus Kuhmilch (Sigma)
o Puffer: 100 mM NaH₂PO₄, 50 mM NaCl, pH 8,5
o Marker A: Alexa Fluor® 488 Cadaverine (Invitrogen)
o Sonstiges: EDC (Merck), Sulfo-NHS (Pierce)

Durchführung: 10 mg Protein (0,54 µmol) wurden in 100 µl Puffer gelöst. Dazu wurden je 10 µl EDC (20 mg/ml; bzw. 1,3 µmol, das ist 2-fach molarer Überschuss bezogen auf das Protein) und Sulfo-NHS (20 mg/ml; bzw. 0,92 µmol, das ist 1-fach molarer Überschuss bezogen auf das Protein) pipettiert. Die Lösung wurde für 4 Stunden bei Raumtemperatur inkubiert, danach komplett in ein Röhrchen Alexa Fluor® 488 Cadaverin (1 mg; bzw.1,56 µmol, das ist 3-fach molarer Überschuss bezogen auf das Protein) überführt. Die Lösung wurde dunkel über Nacht bei Raumtemperatur inkubiert.

### Beispiel für Schritt b: Abtrennen des nichtgebundenen Markers A

Das gebundene Protein wurde durch Größenausschlusschromatographie vom freien Marker A getrennt. Verwendete Materialien:
o Laufmittel: 100 mM NaH₂PO₄, 50 mM NaCl, pH 8,5
o Säule: Superdex® Peptide 10/300 GL Säule (GE Healthcare), die stationäre Phase ist quervernetzte Agarose und Dextran, pH stabil zwischen pH 3 und 12, 10 x 300 mm

Durchführung: 50 µl (ca. 5 mg Protein) der Probe aus Beispiel für Schritt a wurden nach Äquilibrieren der Säule laut Herstellerangaben aufgetragen. Imzuge der fraktionierten Elution mit dem genannten Laufmittel wurde das gekoppelte Protein vom freien Marker A getrennt und damit isoliert (in einem Volumen von ca. 3 ml). Volumenbezogene Proteinkonzentration ca. 1,7 mg/ml.

### Beispiel für Schritt c: Spalten des gekoppelten Protein

Die Spaltung wurde mit Trypsin vorgenommen, welches auf einer Säule immobilisiert war. Verwendete Materialien:
o Säule: Poroszyme® Immobilized Trypsin Cartridge; Säule mit immobilisiertem Trypsin (Applied Biosystems), Polystyroldivinylbenzol Partikel mit Poren zwischen 500 und 10000 Å, 2.1 x 30 mm
o Spaltpuffer: 100 mM NaH₂PO₄, 50 mM NaCl, pH 8,5

Durchführung: Als Vorversuch wurden 100 µl auf die Säule aufgetragen und gespalten. Nach positiver Kontrolle der Spaltung mittels HPLC-Analytik (siehe Beispiel für Schritt e) wurden 1 ml (ca. 1,7 mg) der Probe analog gespalten (gemäß Herstellerangaben). Das gespaltene Material wurde aufgefangen; Volumen ca. 1,2 ml. Volumenbezogene Proteinkonzentration nun 1,39 mg/ml.

### Beispiel für Schritt d1: Kopplung freier COOH-Gruppen mit Marker B

Die nach der Trypsinspaltung aus Schritt c neu frei gewordenen COOH-Gruppen wurden erneut mit einem Marker B markiert. Dies passierte nach Aktivierung der COOH-Gruppen mit EDC/Sulfo-NHS. Verwendete Materialien:
o Puffer: 100 mM NaH₂PO₄, 50 mM NaCl, pH 8,5
o Farbstoff: Alexa Fluor® 350 Cadaverine (Invitrogen)
o Sonstiges: EDC (Merck), Sulfo-NHS (Pierce)

Durchführung: Zu 1 ml der Probe (1,39 mg; bzw. 75,5 nmol) wurden je 0,65 µmol EDC und 0,46 µmol Sulfo-NHS pipettiert. Die Lösung wurde für 4 Stunden bei Raumtemperatur inkubiert, danach komplett in 2,52 µmol Alexa Fluor® 350 Cadaverin überführt. Die Lösung wurde dunkel über Nacht bei Raumtemperatur inkubiert.

### Beispiel für Schritt d2: Abtrennen des nicht gebundenen Farbstoff

Verwendete Materialien:
o Laufmittel: 100 mM NaH₂PO₄, 50 mM NaCl, pH 8,5
o Säule: Superdex® Peptide 10/300 GL (GE Healthcare), die stationäre Phase ist quervernetzte Agarose und Dextran, pH stabil zwischen pH 3 und 12, 10 x 300 mm

Durchführung: 200 µl (ca. 0,28 mg Protein) der Probe wurden nach Äquilibrieren der Säule aufgetragen (gemäß Herstelleranganben). Das gebundene Protein wurde durch Größenausschlusschromatographie vom freien Marker B getrennt. Imzuge fraktionierter Elution wurde das gebundene Protein abgetrennt (ca. 2 ml). Dies wurde wiederholt. Theoretische Proteinkonzentration ca. 0,14 mg/ml.

### Beispiel für Schritt e: HPLC-Analytik

Verwendete Materialien:
o Laufmittel A: Wasser; 0,1 % TFA
o Laufmittel B: Acetonitril; 0,1 % TFA
o Säule: Jupiter 4 µ Proteo 90 Å; 250 x 4,6 mm (Phenomenex), 90 Å Säule, C₁₂ gebundene stationäre Phase, Umkehrphasen-Technologie, Trennung von Peptiden < 10000 MW

Durchführung: Mittels automatisierter Gradientenelution, d.h. Auftragung der Probe mit Laufmittel A und Elution durch gesteigerte Konzentration von Laufmittel B, wurden die einzelnen Peptidfragmente nach Hydrophobizität aufgetrennt, und mittels UV- Detektor und Fluoreszenzdetektor detektiert. Die verwendete HPLC Technik stammt aus der Shimadzu 10A Serie. Das System verfügt über HPLC-Pumpen, deren Steuerung über Software integriert ist. Das System verfügt auch über ein automatisches Probenaufgabensystem und ein integriertes Detektorensystem für UV (SPD 10A) und Fluoreszenzdetektion (RF 10A), welche ein Diodenarray-Detektionssystem sind.

### Beispiel für Schritt f: Sequenzierung des C-Terminus

Nach der Isolierung des Peptides (manuelle Fraktionierung während des RP-HPLC-Laufes) wurde dieses am Edman-Sequenzer (der Firma ABI) nach üblichem Vorgehen analysiert und somit die Aminosäuresequenz festgestellt.

## Patentansprüche

1. Verfahren zur Sequenzierung des C-Terminus eines Proteins, **dadurch gekennzeichnet, dass** man:
a) die freien COOH-Gruppen im Protein mit Marker A umsetzt
b) freien Marker A entfernt
c) das markierte Protein in Peptide spaltet
d) die durch Schritt c neu freigewordenen COOH-Gruppen gegebenenfalls mit Marker B umsetzt, der sich von Marker A unterscheidet
e) das ausschließlich mit Marker A markierte C-terminale Peptidstück identifiziert und
f) das ausschließlich mit Marker A markierte C-terminale Peptidstück in üblicher Weise sequenziert.

2. Verfahren gemäß Anspruch 1, **dadurch gekennzeichnet, dass** man die Kopplung der freien COOH-Gruppen mit den Markern A bzw. B nach Aktivierung dieser COOH-Gruppen vornimmt.

3. Verfahren gemäß Anspruch 2, **dadurch gekennzeichnet, dass** man zur Aktivierung Carbodiimide, gegebenenfalls in Kombination mit N-Hydroxysuccinimid (NHS) verwendet.

4. Verfahren gemäß den Ansprüchen 2 und 3, **dadurch gekennzeichnet, dass** man zur Aktivierung der freien COOH-Gruppen ein Marker-gekoppeltes Aktivierungsreagenz verwendet.

5. Verfahren gemäß einem der vorangegangen Ansprüche, **dadurch gekennzeichnet, dass** man als Marker Fluoreszenzfarbstoffe verwendet.

6. Verfahren gemäß einem der vorangegangen Ansprüche, **dadurch gekennzeichnet, dass** man zur Spaltung des Proteins in Schritt c ein Enzym verwendet.

7. Verfahren gemäß Anspruch 6, **dadurch gekennzeichnet, dass** das Enzym eine Endopeptidase, insbesondere Trypsin oder V8 ist.

8. Verfahren gemäß den Ansprüchen 6 und 7, **dadurch gekennzeichnet, dass** das Enzym auf einer stationären Phase immobilisiert ist.
